# EUROPEAN PATENT SPECIFICATION

(11) **EP 3 083 582 B1**
(45) Date of publication and mention of the grant of the patent: **26.06.2019**
(21) Application number: 14747598.2
(22) Date of filing: 30.07.2014
(51) Int. Cl.: C07D 401/10, C07D 257/04

(54) **A NEW PROCESS FOR THE PREPARATION OF CANDESARTAN CILEXETIL**
NEUES VERFAHREN FÜR DIE ZUBEREITUNG VON CANDESARTAN-CILEXETIL
NOUVEAU PROCÉDÉ POUR LA PRÉPARATION DE CANDÉSARTAN CILEXÉTIL

(30) Priority: 20.12.2013 SI 201300438
(43) Date of publication of application: 26.10.2016
(73) Proprietor: Farma GRS, d.o.o., 8000 Novo mesto (SI)
(72) Inventor: ZUPANCIC, Silvo, 8000 Novo mesto (SI); KRALJ, David, 8210 Trebnje (SI); ZUPET, Rok, 1000 Ljubljana (SI)
(74) Representative: Hoffmann Eitle
(86) International application number: PCT/EP2014/066379
(87) International publication number: WO 2015/090635

(56) References cited:
- EP-A1- 2 502 919
- YONGJUN MAO ET AL: "A novel and practical synthesis of substituted 2-ethoxy benzimidazole: candesartan cilexetil", HETEROCYCLES : AN INTERNATIONAL JOURNAL FOR REVIEWS AND COMMUNICATIONS IN HETEROCYCLIC CHEMISTRY, JAPAN INSTITUTE OF HETEROCYCLIC CHEMISTRY, JP, vol. 81, no. 6, 7 April 2010 (2010-04-07), pages 1503-1508, XP009180251, ISSN: 0385-5414 cited in the application
- MÁRTA PORCS-MAKKAY ET AL: "New Practical Synthesis of the Key Intermediate of Candesartan", ORGANIC PROCESS RESEARCH & DEVELOPMENT, vol. 11, no. 3, May 2007 (2007-05), pages 490-493, XP055141741, ISSN: 1083-6160, DOI: 10.1021/op700041z

## Description

### Field of the invention

The present invention relates to an improved process for the manufacture of candesartan and pharmaceutically acceptable salts and esters thereof and to intermediate products useful in this process.

### Background of the invention

Candesartan cilexetil of formula (I) is chemically described as (+/-)-1-[[(cyclohexyloxy)carbonyl]oxy]ethyl-2-ethoxy-1-[[2'-(1*H*-tetrazol-5-yl)-1,1'-biphenyl-4-yl]methyl]-1*H*-benzimidazole-7-carboxylate. Candesartan is a selective AT1 subtype angiotensin II receptor antagonist. Because of its ability to inhibit the angiotensin-converting enzyme it is widely used for the treatment of hypertension and related diseases and conditions.

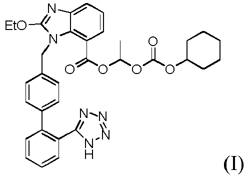

Processes for the preparation of Candesartan cilexetil are well known and disclosed in e.g. WO 2013/041944, WO 2011/145100, WO 2011/080684, WO 2011/061996, WO 2010/067913, WO 2010/034118, WO 2009/157001, WO 2009/115585.

CN 101646659C relates to the process of production of intermediate in the synthesis of candesartan cilexetil with chemical name 1-(((cyclohexyloxy)carbonyl)oxy)ethyl 2-((tert-butoxycarbonyl)((2'-(1-trityl-1H-tetrazol-5-yl)-[1,1'-biphenyl]-4-yl)methyl)amino)-3-nitrobenzoate.

Yongjun Mao, Heterocycles, Vol. 81, No. 6, 2010, p. 1503-1508 discloses the process for the production of candesartan cilexetil wherein the intermediate compounds with chemical name 1-(((cyclohexyloxy)carbonyl)oxy)ethyl 2-((tert-butoxycarbonyl)((2'-(1-trityl-1H-tetrazol-5-yl)-[1,1'-biphenyl]-4-yl)methyl)amino)-3-nitrobenzoate and 1-(((cyclohexyloxy)carbonyl)oxy)ethyl 2-(((2'-(1H-tetrazol-5-yl)-[1,1'-biphenyl]-4-yl)methyl)amino)-3-nitrobenzoate are purified by using chromatography on silica gel. Moreover, in the step of obtaining 1-(((cyclohexyloxy)carbonyl)oxy)ethyl 2-(((2'-(1H-tetrazol-5-yl)-[1,1'-biphenyl]-4-yl)methyl)amino)-3-aminobenzoate stannous chloride dehydrate is used.

Regardless of these various art known preparation processes, there still exists a need for an efficient synthesis of candesartan cilexetil which provides for mild reaction conditions and which does not require further cumbersome purification steps, such as chromatography in particular.

Therefore, the goal of the present invention is a novel process for the preparation of candesartan cilexetil with high yield on industrial level without using hazardous organotin compounds while omitting purification of intermediates by column chromatography.

### Summary of the invention

The present invention relates to a process for the preparation of candesartan cilexetil comprising the steps of:
a) hydrogenation of 1-(((cyclohexyloxy)carbonyl)oxy)ethyl 2-((*tert*-butoxycarbonyl)((2'-(1-trityl-1*H*-tetrazol-5-yl)-[1,1'-biphenyl]-4-yl)methyl)amino)-3-nitrobenzoate by using a catalyst in an organic solvent to get an oily product of 1-(((cyclohexyloxy)carbonyl)oxy)ethyl 3-amino-2-((tert-butoxycarbonyl)((2'-(1-trityl-1H-tetrazol-5-yl)-[1,1'-biphenyl]-4-yl)methyl)amino)benzoate, wherein the catalyst is Raney-Ni,
b) addition of an acid in an organic solvent to get 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, wherein the acid is HCl,
c) precipitation of 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, optionally by addition of an anti-solvent selected from the group consisting of an ether, an alkane, a chlorinated alkane or any mixture thereof,
d) ring closure with tetraethyl orthocarbonate to get candesartan cilexetil,
e) optional addition of an anti-solvent, wherein the anti-solvent is selected from the group consisting of an ether such as THF, *tert*-butyl methyl ether, methyl THF, diisopropyl ether and dioxane, an ester such as ethyl acetate and isopropyl acetate or any mixture thereof.

In addition, the present invention relates to a process for the preparation of candesartan cilexetil comprising the steps of:
a') reaction between C₁₋₄alkyl 2-((*tert*-butoxycarbonyl)amino)-3-nitrobenzoate and 5-(4'-(bromomethyl)-[1,1'-biphenyl]-2-yl)-1-trityl-1H-tetrazole to give C₁₋₄alkyl 2-((tert-butoxycarbonyl)((2'-(1-trityl-1H-tetrazol-5-yl)-[1,1'-biphenyl]-4-yl)methyl)amino)-3-nitrobenzoate,
a") hydrolysis of C₁₋₄alkyl 2-((tert-butoxycarbonyl)((2'-(1-trityl-1H-tetrazol-5-yl)-[1,1'-biphenyl]-4-yl)methyl)amino)-3-nitrobenzoate and reaction to give 1-(((cyclohexyloxy)carbonyl)oxy)ethyl 2-((tert-butoxycarbonyl)((2'-(1-trityl-1H-tetrazol-5-yl)-[1,1'-biphenyl]-4-yl)methyl)amino)-3-nitrobenzoate,
a) hydrogenation of 1-(((cyclohexyloxy)carbonyl)oxy)ethyl 2-((*tert*-butoxycarbonyl)((2'-(1-trityl-1*H*-tetrazol-5-yl)-[1,1'-biphenyl]-4-yl)methyl)amino)-3-nitrobenzoate by using a catalyst in an organic solvent to get an oily product of 1-(((cyclohexyloxy)carbonyl)oxy)ethyl 3-amino-2-((tert-butoxycarbonyl)((2'-(1-trityl-1H-tetrazol-5-yl)-[1,1'-biphenyl]-4-yl)methyl)amino)benzoate as specified in appended claim 1,
b) addition of an acid in an organic solvent to get 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, wherein the acid is HCl,
c) precipitation of 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, optionally by addition of an anti-solvent,
d) ring closure with tetraethyl orthocarbonate to get candesartan cilexetil,
e) optional addition of an anti-solvent.

In a preferred embodiment, the present invention relates to a process for the preparation of candesartan cilexetil comprising the steps of:
a') reaction between ethyl 2-((*tert*-butoxycarbonyl)amino)-3-nitrobenzoate and 5-(4'-(bromomethyl)-[1,1'-biphenyl]-2-yl)-1-trityl-1H-tetrazole to give ethyl 2-((tert-butoxycarbonyl)((2'-(1-trityl-1H-tetrazol-5-yl)-[1,1'-biphenyl]-4-yl)methyl)amino)-3-nitrobenzoate,
a") hydrolysis of ethyl 2-((tert-butoxycarbonyl)((2'-(1-trityl-1H-tetrazol-5-yl)-[1,1'-biphenyl]-4-yl)methyl)amino)-3-nitrobenzoate to give 1-(((cyclohexyloxy)carbonyl)oxy)ethyl 2-((tert-butoxycarbonyl)((2'-(1-trityl-1H-tetrazol-5-yl)-[1,1'-biphenyl]-4-yl)methyl)amino)-3-nitrobenzoate,
a) hydrogenation of 1-(((cyclohexyloxy)carbonyl)oxy)ethyl 2-((*tert*-butoxycarbonyl)((2'-(1-trityl-1*H*-tetrazol-5-yl)-[1,1'-biphenyl]-4-yl)methyl)amino)-3-nitrobenzoate by using a catalyst in an organic solvent to get an oily product of 1-(((cyclohexyloxy)carbonyl)oxy)ethyl 3-amino-2-((tert-butoxycarbonyl)((2'-(1-trityl-1H-tetrazol-5-yl)-[1,1'-biphenyl]-4-yl)methyl)amino)benzoate as specified in appended claim 1,
b) addition of an acid in an organic solvent to get 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, wherein the acid is HCl,
c) precipitation of 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, optionally by addition of an anti-solvent,
d) ring closure with tetraethyl orthocarbonate to get candesartan cilexetil,
e) optional addition of an anti-solvent.

In another embodiment the present invention relates to compound 1-(((cyclohexyloxy)carbonyl)oxy)ethyl 3-amino-2-((*tert*-butoxycarbonyl)((2'-(1-trityl-1H-tetrazol-5-yl)-[1,1'-biphenyl]-4-I)methyl)amino)benzoate or any pharmaceutically acceptable salt thereof.

In yet another embodiment the present invention relates to compound 1-(((cyclohexyloxy)carbonyl)oxy)ethyl 2-(((2'-(1*H*-tetrazol-5-yl)-[1,1'-biphenyl]-4-yl)methyl)amino)-3-aminobenzoate in the form of hydrochloride salt.

In yet another embodiment the present invention relates to a process for the preparation of a pharmaceutical composition comprising the steps of preparing candesartan cilexetil by a process according to any of the appended claims 1 to 9 followed by mixing a therapeutically effective amount thereof with one or more pharmaceutically acceptable excipient(s) and optionally with one or more other active substance(s).

### Detailed description of the invention

The present inventors surprisingly found that by hydrogenation of NO₂ group of 1-(((cyclohexyloxy)carbonyl)oxy)ethyl 2-((*tert*-butoxycarbonyl)((2'-(-trityl-1*H*-tetrazol-5-yl)-[1,1'-biphenyl]-4-yl)methyl)amino)-3-nitrobenzoate by using a catalyst in an organic solvent, subsequent cleavage of the *N*-Boc group under acidic condition, followed by isolation of solid 1-(((cyclohexyloxy)carbonyl)oxy)ethyl 2-(((2'-(1*H*-tetrazol-5-yl)-[1,1'-biphenyl]-4-yl)methyl)amino)-3-aminobenzoate in the form of any pharmaceutically acceptable salt and ring closure by using tetraethyl orthocarbonate in an organic solvent to obtain the final product candesartan cilexetil, the reaction proceeds very well under very mild conditions. The process does not require cumbersome purification steps such as column chromatography and it gives the final product with high yield and purity. The process can be easily used on industrial level.

Therefore, the first embodiment of the present invention is a process for the preparation of candesartan cilexetil comprising the steps of:
a) hydrogenation of 1-(((cyclohexyloxy)carbonyl)oxy)ethyl 2-((*tert*-butoxycarbonyl)((2'-(1-trityl-1*H*-tetrazol-5-yl)-[1,1'-biphenyl]-4-yl)methyl)amino)-3-nitrobenzoate by using a catalyst in an organic solvent to get 1-(((cyclohexyloxy)carbonyl)oxy)ethyl 3-amino-2-((tert-butoxycarbonyl)((2'-(1-trityl-1H-tetrazol-5-yl)-[1,1'-biphenyl]-4-yl)methyl)amino)benzoate, wherein the catalyst is Raney-Ni,
b) addition of an acid in an organic solvent to get 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, wherein the acid is HCl,
c) precipitation of 1-(((cyclohexyloxy)carbonyl)oxy)ethyl 2-(((2'-(1H-tetrazol-5-yl)-[1,1 biphenyl]-4-yl)methyl)amino)-3-aminobenzoate in the form of any pharmaceutically acceptable salt, optionally by addition of an anti-solvent selected from the group consisting of an ether, an alkane, a chlorinated alkane or any mixture thereof,
d) ring closure with tetraethyl orthocarbonate to get candesartan cilexetil,
e) optional addition of an anti-solvent, wherein the anti-solvent is selected from the group consisting of an ether such as THF, *tert*-butyl methyl ether, methyl THF, diisopropyl ether and dioxane, an ester such as ethyl acetate and isopropyl acetate or any mixture thereof.

The route of the process of the present invention is presented in Scheme 1.

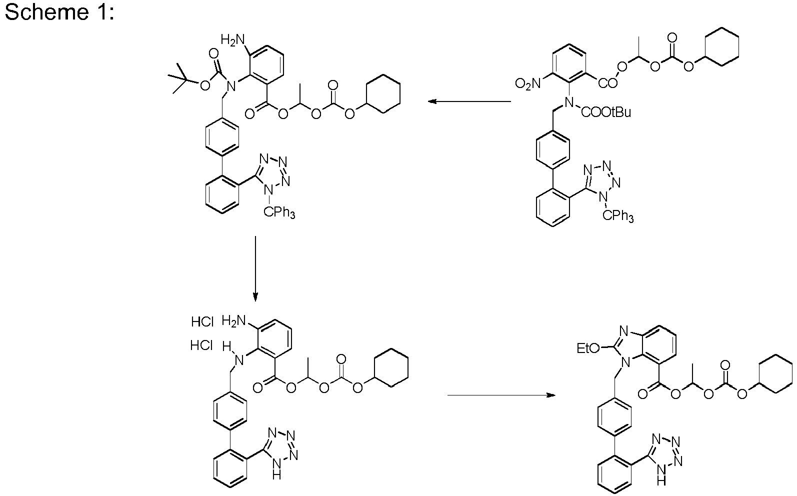

Easy performed crystallization of the key intermediate in the synthesis 1-(((cyclohexyloxy)carbonyl)oxy)ethyl 2-(((2'-(1H-tetrazol-5-yl)-[1,1'-biphenyl]-4-yl)methyl)amino)-3-aminobenzoate or its pharmaceutically acceptable salt from an appropriate solvent or a solvent mixture already provides the desired intermediate in a high yield and purity. Moreover, in comparison to the known prior art process such as disclosed by Mao, Yongjun, Heterocycles, Volume: 81, Issue: 6, Pages: 1503-1508, the use of stannous chloride is not required.

Accordingly, the present invention relates to a novel processes for the preparation of candesartan cilexetil and reaction intermediates suitable for manufacturing the same.

According to the present invention, the obtained candesartan cilexetil can be in the form of the amorphous substance or any crystalline (polymorphic) form thereof. Moreover, it is contemplated in the context of the present invention to prepare any pharmaceutically acceptable solvate (hydrate) form of candesartan cilexetil.

According to one embodiment of the invention the inventive process comprises the step of hydrogenation of 1-(((cyclohexyloxy)carbonyl)oxy)ethyl 2-((*tert*-butoxycarbonyl)((2'-(1-trityl-1*H*-tetrazol-5-yl)-[1,1'-biphenyl]-4-yl)methyl)amino)-3-nitrobenzoate by using a catalyst in an organic solvent, wherein the catalyst is Raney-Ni. The organic solvent can be an alcohol, preferably an alcohol consisting of a linear or branched C₁ to C₁₀ alkyl group to which a hydroxyl group is attached, an ester, preferably an ester of the general type R¹-COO-R² with R¹ and R² each independently representing a linear or branched C₁ to C₁₀ alkyl group, such as ethyl acetate, or any mixture thereof, preferably isopropyl acetate is used. The hydrogenation is performed at temperature below 50°C, preferably at room temperature.

If isolated, the obtained product is typically an oily substance. It may be further processed as such. However, it is also possible to further process the product also in other forms, e.g. in dissolved form.

To the obtained product 1-(((cyclohexyloxy)carbonyl)oxy)ethyl 3-amino-2-((tert-butoxycarbonyl)((2'-(1-trityl-1H-tetrazol-5-yl)-[1,1'-biphenyl]-4-yl)methyl)amino)benzoate an acid is added, preferably in an organic solvent. The acid is HCl. Acid present in the reaction mixture acts as a reagent for deprotection of protecting groups and for formation of a salt which precipitates from the reaction mixture. The reaction is performed at temperature below 40°C, preferably at a temperature of from 15°C to 30°C and more preferably at room temperature such as 20°C to 25°C. It is preferably performed in the presence of an organic solvent. The organic solvent can be an alcohol, ester or ether, or any mixture thereof. Preferably, the alcohol consists of a linear or branched C₁ to C₁₀ alkyl group to which a hydroxyl group is attached, the ester preferably is an ester of the general type R¹-COO-R² with R¹ and R² each independently representing a linear or branched C₁ to C₁₀ alkyl group, and the ether is preferably an ether of the general type R¹-O-R² with R¹ and R² each independently representing a linear or branched C₁ to C₁₀ alkyl group or R¹ and R² being alkyl groups connected to each other to form a 5 or 6-membered cycle. More preferably ethanol is used. After the reaction is completed 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt is precipitated as a solid product from the reaction mixture. The solid product is preferably HCl salt.

Before, during or after the product is precipitated an anti-solvent may be added, the anti-solvent can be selected from group consisting of an ether, an alkane, a chlorinated alkane, or any mixture thereof. Preferably, the ether is an ether of the general type R¹-O-R² with R¹ and R² each independently representing a linear or branched C₁ to C₁₀ alkyl group or R¹ and R² being alkyl groups connected to each other to form a 5 or 6-membered cycle. Preferably, the alkane is a linear or branched alkane with 5 to 10 carbon atoms. The chlorinated alkane is preferably a linear or branched alkane with 1 to 10 carbon atoms and one or more chlorine atoms, provided that the number of carbons and the number of chlorine atoms is selected such that the substance is liquid under normal pressure and at 20°C. More preferably *tert-*butyl methyl ether can be used as anti-solvent.

A salt of 1-(((cyclohexyloxy)carbonyl)oxy)ethyl 2-(((2'-(1H-tetrazol-5-yl)-[1,1'-biphenyl]-4-yl)methyl)amino)-3-aminobenzoate is well suited for purification. During the synthesis byproducts are formed, said byproducts are removed during the crystallization process.

The obtained salt is further processed to candesartan cilexetil by ring closure with tetraethyl orthocarbonate. In the present invention molar ratio between 1-(((cyclohexyloxy)carbonyl)oxy)ethyl 2-(((2'-(1H-tetrazol-5-yl)-[1,1'-biphenyl]-4-yl)methyl)amino)-3-aminobenzoate and tetraethyl orthocarbonate can be from 1 : 3 to 1 : 8, preferably 1 : 3 to 1 : 6, most preferably from 1 : 3 to 1 : 5. The temperature of the reaction is below 40°C, preferably between 0°C and 25°C, most preferably between 0°C and 10°C. An acid selected from the group of an organic acid might optionally be added, but preferably no acid is added. The reaction may be performed without solvent or it is performed with any solvent, preferably the solvent such as for example an organic solvent selected from the group consisting of an ether such as for example THF, *tert-butyl* methyl ether, methyl THF, diisopropyl ether, dioxane, an ester such as for example ethyl acetate, isopropyl acetate, dichloromethane, or any mixture thereof, preferably THF is used. The volume ratio between tetraethyl orthocarbonate and the solvent is from 1: 0 to 1 : 2, preferably from 1: 0 to 1 : 1, most preferably from 1 : 0 to 1 : 0.5. After the reaction is completed the product precipitates and an anti-solvent may be added before, during or after precipitation. The anti-solvent may be selected from the group consisting of an ether. The ether is preferably an ether of the general type R¹-O-R² with R¹ and R² each independently representing a linear or branched C₁ to C₁₀ alkyl group or R¹ and R² being alkyl groups connected to each other to form a 5 or 6-membered cycle, such as for example THF, *tert*-butyl methyl ether, methyl THF, diisopropyl ether and dioxane. The ester is preferably an ester of the general type R¹-COO-R² with R¹ and R² each independently representing a linear or branched C₁ to C₁₀ alkyl group, such as for example ethyl acetate and isopropyl acetate, or any mixture thereof. Preferably *tert*-butyl methy ether or THF can be used.

Another embodiment of the present invention relates to methods for the preparation of candesartan cilexetil, comprising the above process as reaction steps.

A further preferred embodiment relates to a novel intermediate in the process for the preparation of candesartan cilexetil with chemical name 1-(((cyclohexyloxy)carbonyl)oxy)ethyl 3-amino-2-((*tert-*butoxycarbonyl)((2'-(1-trityl-1H-tetrazol-5-yl)-[1,1'-biphenyl]-4-I)methyl)amino)benzoate or any pharmaceutically acceptable salt thereof.

Yet another preferred embodiment relates to a novel intermediate in the process for the preparation of candesartan cilexetil with chemical name 1-(((cyclohexyloxy)carbonyl)oxy)ethyl 2-(((2'-(1*H*-tetrazol-5-yl)-[1,1'-biphenyl]-4-yl)methyl)amino)-3-aminobenzoate in the form of a hydrochloride salt. The hyrochloride salt can be monohydrochloride or dihydrochloride.

By formation and precipitation of 1-(((cyclohexyloxy)carbonyl)oxy)ethyl 2-(((2'-(1*H*-tetrazol-5-yl)-[1,1'-biphenyl]-4-yl)methyl)amino)-3-aminobenzoate in the form of any pharmaceutically acceptable salt all impurities and byproducts originating from all previous steps are removed and consequently no additional purification is required and needed (as e.g. column chromatography).

Another embodiment the present invention relates to a novel process for the preparation of candesartan cilexetil comprising the reaction between the commercially available ethyl 2-((*tert*-butoxycarbonyl)amino)-3-nitrobenzoate and the commercially available 5-(4'-(bromomethyl)-[1,1'-biphenyl]-2-yl)-1-trityl-1H-tetrazole to give the intermediate with chemical name ethyl 2-((tert-butoxycarbonyl)((2'-(1-trityl-1H-tetrazol-5-yl)-[1,1'-biphenyl]-4-yl)methyl)amino)-3-nitrobenzoate which upon hydrolysis and reaction gives 1-(((cyclohexyloxy)carbonyl)oxy)ethyl 2-((tert-butoxycarbonyl)((2'-(1-trityl-1H-tetrazol-5-yl)-[1,1'-biphenyl]-4-yl)methyl)amino)-3-nitrobenzoate that is hydrogenated by using a catalyst in an organic solvent to yield the oily product 1-(((cyclohexyloxy)carbonyl)oxy)ethyl 3-amino-2-((tert-butoxycarbonyl)((2'-(1-trityl-1H-tetrazol-5-yl)-[1,1'-biphenyl]-4-yl)methyl)amino)benzoate or any pharmaceutically acceptable salt thereof. The oily product is converted to 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt which is recrystallized from a suitable solvent and subsequent converted into candesartan cilexetil. In this process, it is also possible to use another C₁₋₄alkyl ester instead of the ethyl ester in the first two reaction steps.

Therefore, next embodiment of the present invention is a process for the preparation of candesartan cilexetil comprising the steps of:
a') reaction between C₁₋₄alkyl 2-((*tert*-butoxycarbonyl)amino)-3-nitrobenzoate and 5-(4'-(bromomethyl)-[1,1'-biphenyl]-2-yl)-1-trityl-1H-tetrazole to give C₁₋₄alkyl 2-((tert-butoxycarbonyl)((2'-(1-trityl-1H-tetrazol-5-yl)-[1,1'-biphenyl]-4-yl)methyl)amino)-3-nitrobenzoate,
a") hydrolysis of C₁₋₄alkyl 2-((tert-butoxycarbonyl)((2'-(1-trityl-1H-tetrazol-5-yl)-[1,1'-biphenyl]-4-yl)methyl)amino)-3-nitrobenzoate and reaction to give 1-(((cyclohexyloxy)carbonyl)oxy)ethyl 2-((tert-butoxycarbonyl)((2'-(1-trityl-1H-tetrazol-5-yl)-[1,1'-biphenyl]-4-yl)methyl)amino)-3-nitrobenzoate,
a) hydrogenation of 1-(((cyclohexyloxy)carbonyl)oxy)ethyl 2-((*tert*-butoxycarbonyl)((2'-(1-trityl-1*H*-tetrazol-5-yl)-[1,1'-biphenyl]-4-yl)methyl)amino)-3-nitrobenzoate by using a catalyst in an organic solvent to get 1-(((cyclohexyloxy)carbonyl)oxy)ethyl 3-amino-2-((tert-butoxycarbonyl)((2'-(1-trityl-1H-tetrazol-5-yl)-[1,1'-biphenyl]-4-yl)methyl)amino)benzoate as specified in appended claim 1,
b) addition of an acid in an organic solvent to get 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, wherein the acid is HCl,
c) precipitation of 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, optionally by addition of an anti-solvent,
d) ring closure with tetraethyl orthocarbonate to get candesartan cilexetil,
e) optional addition of an anti-solvent.

The route of the process of the present invention is presented in Scheme 2.

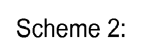

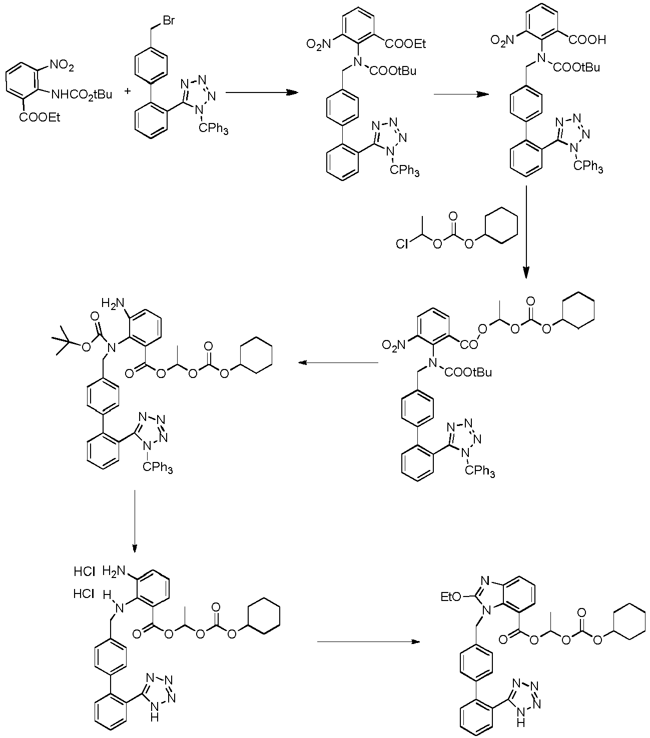

The above reaction scheme shows 1-chloroethyl cyclohexyl carbonate as a suitable and preferred reagent for converting ethyl 2-((tert-butoxycarbonyl)((2'-(1-trityl-1H-tetrazol-5-yl)-[1,1'-biphenyl]-4-yl)methyl)amino)-3-nitrobenzoate after the hydrolysis to give 1-(((cyclohexyloxy)carbonyl)oxy)ethyl 2-((tert-butoxycarbonyl)((2'-(1-trityl-1H-tetrazol-5-yl)-[1,1'-biphenyl]-4-yl)methyl)amino)-3-nitrobenzoate. However, in other embodiments the reagent may also carry a leaving group different from chlorine.

Moreover, the above reaction scheme shows the use of the ethyl ester in the first two reaction steps. As noted above, it is also possible to use another C₁₋₄alkyl ester.

In a further preferred embodiment of the present invention the obtained candesartan cilexetil has a purity of at least 97%, preferably of at least 98% and more preferably of at least 99%.

The yield of the complete synthesis from ethyl 2-((*tert*-butoxycarbonyl)amino)-3-nitrobenzoate to crude candesartan cilexetil is at least 60%, preferably at least 65%.

The yield of the synthesis from 1-(((cyclohexyloxy)carbonyl)oxy)ethyl 2-((*tert-*butoxycarbonyl)((2'-(1-trityl-1*H*-tetrazol-5-yl)-[1,1'-biphenyl]-4-yl)methyl)amino)-3-nitrobenzoate to crude candesartan cilexitil is typically greater than 85%, preferably between 95 and 99%, more preferably between 97 and 99%.

Another embodiment of the present invention relates to methods for the preparation of candesartan cilexetil, comprising the above process as a reaction step.

In another embodiment the present invention relates to a process for the preparation of a pharmaceutical composition comprising the steps of preparing candesartan cilexetil according to the above processes and mixing a therapeutically effective amount thereof with one or more pharmaceutically acceptable excipients and optionally with one or more further active substances. Preferred examples of such further active substances are selected from the group of antihypertensives that can be selected from the group of angiotensive converting enzyme (ACE) inhibitors, angiotensin receptor blockers (ARBs), calcium channel blockers (CCBs) and antiadrenergics. Lipid regulators can for example be HMG CoA reductase inhibitors, diuretics such as hydrochlorothiazide (HCTZ) or furosemide, lipid regulators or antidiabetics. According to the present invention, HCTZ preferably has an average particle size of less than 80 µm, preferably less than 50 µm, more preferably less than 30 µm.

The term "average particle size" as used herein refers to the volume mean diameter of the particles. The volume mean diameter was determined by laser light scattering using a Malvern-Mastersizer Apparatus MS 2000 equiped with Hydro S dispersion unit. Vegetable oil was used as the dilution medium.

A therapeutically effective amount of candesartan cilexetil is preferably provided in a unit dosage form, each unit dosage form containing from about 1-100 mg, preferably 1-50 mg, and even more preferably 4-32 mg of candesartan cilexetil.

It has surprisingly been found that the stability of the candesartan cilexetil composition according to the invention is not adversely affected if the other active ingredient is selected from the group consisting of captopril, enalapril, cilazapril, lisinopril, trandolapril, ramipril, fosinopril, perindopril, amlodipine, diltiazem, felodipine, nifedipine, nitrendipine, verapamil, acebutol, atenolol, betaxolol, bisoprolol, metoprolol, carvedilol, lovastatin, simvastatin, pravastatin, atorvastatin, fluvastatin, rosuvastatin, indapamide, hydrochlorothiazide, sulfonyl urea, metglinides such as nateglinide or repaglinide, thiazolidinediones such as pioglitazone or rosiglitazone, α-glucosidase inhibitors, incretin mimetics, biguanides such as metformin hydrochloride and pharmaceutically acceptable salts thereof.

The pharmaceutically acceptable excipients used in and for the preparation of the present pharmaceutical composition particularly include diluents, binders, disintegrants and lubricants. Other and further excipients can also be contained.

The pharmaceutical composition can be used for oral administration such as in the form of tablets, coated tablets, capsules, sachet and oral liquids, or can be used for topical administration such as in the form of gels, lotions, patches, or ointments, or can be used for parenteral administration such as intravenous, intramuscular, or subcutaneous injection, or can be used as suppositories.

The present invention is described in more detail by the following examples, but is not limited thereto.

### Description of HPLC method:

### Equipment

HPLC: Agilent 1100
Data evaluation: ChemStation Chromatographic conditions:
Column: Zorbax Eclipse XDB C- 18, 1,8 µm, 50 x 4,6 mm

### Mobile Phase:

Solvent A: 0,0IM sodium dihydrogenphosphate, pH 2.5
Solvent B: acetonitrile

### Gradient:

| Time (min) | % A | % B |
|---|---|---|
| 0 | 55 | 45 |
| 16 | 5 | 95 |
| 18 | 5 | 95 |
| 19 | 55 | 45 |

Post run: 2 min
Column temp, 30°C
Flow rate: 1.0 ml/min
Detection: UV, 225nm
Injection: 10 µl

### Example 1: Ethyl 2-((tert-butoxycarbonyl)((2'-(1-trityl-1H-tetrazol-5-yl)-[1,1'-biphenyl]-4-yl)methyl)amino)-3-nitrobenzoate

The mixture of 4.68g (15mmol) of ethyl 2-((*tert*-butoxycarbonyl)amino)-3-nitrobenzoate, 8.75g (15.7 mmol) of 5-(4'-(bromomethyl)-[1,1'-biphenyl]-2-yl)-1-trityl-1*H*-tetrazole, 4.64 g (33.6 mmol) of K₂CO₃ and 60.3 ml of acetonitrile is heated to reflux temperature (aprox. 80°C) for 7 hours. The mixture is cooled and filtered. Filter cake is washed with acetonitrile (2x 50ml). The filtrate is concentrated to thick oil (title compound m = 13.3g).

HPLC purity: 90.1% of the title product

### Example 2: 2-((tert-Butoxycarbonyl)((2'-(1-trityl-1H-tetrazol-5-yl)-[1,1'-biphenyl]-4-yl)methyl)amino)-3-nitrobenzoic acid

13.3g of Ethyl 2-((*tert*-butoxycarbonyl)((2'-(1-trityl-1*H*-tetrazol-5-yl)-[1,1'-biphenyl]-4-yl)methyl)amino)-3-nitrobenzoate (the product of previous reaction) is dissolved in 56 ml of DMA and then 15.6 ml of 6M NaOH is added. The mixture is stirred for 2 hours at room temperature. Then the reaction mixture is neutralized to pH 2-3. During the neutralization 94 ml of isopropyl acetate is added. After the neutralization 62 ml of water is added and the phases are separated. Organic phase is then washed with water (2× with 62 ml) and concentrated to the oily residue (title compound 14.4 g).

HPLC purity: 89.4% of the title product

### Example 3: 1-(((Cyclohexyloxy)carbonyl)oxy)ethyl 2-((tert-butoxycarbonyl)((2'-(1-trityl-1H-tetrazol-5-yl)-[1,1'-biphenyl]-4-yl)methyl)amino)-3-nitrobenzoate

The mixture of 13.4 g of 2-((*tert*-Butoxycarbonyl)((2'-(1-trityl-1*H*-tetrazol-5-yl)-[1,1'-biphenyl]-4-yl)methyl)amino)-3-nitrobenzoic acid (the product of previous reaction), 11.2 ml of *N*,*N-*dimethylacetamide, 4.06 g of 1-chloroethyl cyclohexyl carbonate and 2.35 g of K₂CO₃ is heated at 60°C for 6 hours. Then the reaction mixture is cooled 82 ml of isopropyl acetate and 82 ml of water are added. The phases are separated and aqueous phase is reextracted with 82 ml of isopropyl acetate. Collected organic phases are washed with water (2 × 82 ml). Isolated organic phase is a solution of the title product in isopropyl acetate.

HPLC purity: 87.5% of the title product

### Example 4: 1-(((Cyclohexyloxy)carbonyl)oxy)ethyl 3-amino-2-((tert-butoxycarbonyl)((2'-(1-trityl-1H-tetrazol-5-yl)-[1,1'-biphenyl]-4-yl)methyl)amino)benzoate

½ of the volume of the solution of 1-(((Cyclohexyloxy)carbonyl)oxy)ethyl 2-((*tert-*butoxycarbonyl)((2'-(1-trityl-1*H*-tetrazol-5-yl)-[1,1'-biphenyl]-4-yl)methyl)amino)-3-nitrobenzoate (the product of previous reaction) is concentrated to around 50ml and poured into the reactor. The catalyst is added (2.1 g of RaNi) and the mixture is hydrogenated at 3.5 barg and room temperature for 24h. After the reaction is completed the catalyst is filtered of and the solution is concentrated to yield 9.39 g of oily title product.

HPLC purity: 90.7% of the title product

### Example 5: 1-(((cyclohexyloxy)carbonyl)oxy)ethyl 2-(((2'-(1H-tetrazol-5-yl)-[1,1'-biphenyl]-4-yl)methyl)amino)-3-aminobenzoate dihydrochloride

To the 1/3 of the oily product of previous reaction (1-(((Cyclohexyloxy)carbonyl)oxy)ethyl 3-amino-2-((*tert*-butoxycarbonyl)((2'-(1-trityl-1*H*-tetrazol-5-yl)-[1,1'-biphenyl]-4-yl)methyl)amino)benzoate; 3.13g) 9.5 ml of of 3.02 M HCl in Ethanol is added and stirred at room temperature for 3 hours to complete the reaction. Then 85 ml of *tert*-butyl methyl ether is added and the mixture is stirred for 18 hours. The product is filtered off and the product is washed with 4 ml of *tert*-butyl methy ether and dried.

HPLC purity: 94.2% of title product.
m (of title product)=1.25g
Yield: 75% calculate to starting material ethyl 2-((*tert*-butoxycarbonyl)amino)-3-nitrobenzoate.
T (melting point) = 129-136°C
FTIR = 3411, 2938, 2862, 1758, 1708, 1289, 1256, 1072, 762

### Example 6: Candesartan cilexetil

The mixture of 1.87 ml of tetraethyl orthocarbonate, 0.6 ml of THF is cooled to 5°C. Then 1.25g of 1-(((cyclohexyloxy)carbonyl)oxy)ethyl 2-(((2'-(1*H*-tetrazol-5-yl)-[1,1'-biphenyl]-4-yl)methyl)amino)-3-aminobenzoate dihydrochloride is added and the mixture is stirred under inert atmosphere at 5°C for 6 hours. Then 2.4 ml of *tert*-butyl methyl ether is added and the suspension is stirred at 5°C for 6 hours.
HPLC purity: 97%
m = 1.00g
Yield: 83%, from starting material ethyl 2-((*tert*-butoxycarbonyl)amino)-3-nitrobenzoate the yield is 66%.

### Example 7: Methyl 2-((tert-butoxycarbonyl)((2'-(1-trityl-1H-tetrazol-5-yl)-[1,1'-biphenyl]-4-yl)methyl)amino)-3-nitrobenzoate

The mixture of 14.8 (50 mmol) of methyl 2-((tert-butoxycarbonyl)amino)-3-nitrobenzoate, 29 g (52 mmol) of 5-(4'-(bromomethyl)-[1,1'-biphenyl]-2-yl)-1-trityl-1H-tetrazole, 14 g (101 mmol) of K₂CO₃ and 200 ml of acetonitrile is heated to reflux temperature (aprox. 80°C) for 12 hours. The mixture is cooled for about 20°C and filtered. Filter cake is washed with acetonitrile (2x 50ml). The filtrate is concentrated (at temperature up to 50°C) to thick oil and then 150 ml of isopropanol is added and the mixture is heated to reflux temperature to obtain solution. From the solution soon precipitates the product. The suspension is cooled to room temperature in around 1 h and stirred at this temperature for 1 h. The product is filtered and washed with fresh isopropanol (2×25 ml) and dried.
m = 40g
HPLC purity: 93.7% of the title product
Melting point: 163-169.3°C
Yield calculated on pure product: 96%

## Claims

1. A process for the preparation of candesartan cilexetil comprising the steps of:
a) hydrogenation of 1-(((cyclohexyloxy)carbonyl)oxy)ethyl 2-((*tert*-butoxycarbonyl)((2'-(1-trityl-1*H*-tetrazol-5-yl)-[1,1'-biphenyl]-4-yl)methyl)amino)-3-nitrobenzoate by using a catalyst in an organic solvent to get a typically oily product of 1-(((cyclohexyloxy)carbonyl)oxy)ethyl 3-amino-2-((tert-butoxycarbonyl)((2'-(1-trityl-1H-tetrazol-5-yl)-[1,1'-biphenyl]-4-yl)methyl)amino)benzoate, wherein the catalyst is Raney-Ni,
b) addition of an acid in an organic solvent to get 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, wherein the acid is HCl,
c) precipitation of 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, optionally by addition of an anti-solvent selected from the group consisting of an ether, an alkane, a chlorinated alkane or any mixture thereof,
d) ring closure with tetraethyl orthocarbonate to get candesartan cilexetil,
e) optional addition of an anti-solvent, wherein the anti-solvent is selected from the group consisting of an ether such as THF, *tert*-butyl methyl ether, methyl THF, diisopropyl ether and dioxane, an ester such as ethyl acetate and isopropyl acetate or any mixture thereof.

2. A process for the preparation of candesartan cilexetil according to claim 1, wherein 1-(((cyclohexyloxy)carbonyl)oxy)ethyl 2-((tert-butoxycarbonyl)((2'-(1-trityl-1H-tetrazol-5-yl)-[1,1'-biphenyl]-4-yl)methyl)amino)-3-nitrobenzoate is prepared by hydrolysis of ethyl 2-((tert-butoxycarbonyl)((2'-(1-trityl-1H-tetrazol-5-yl)-[1,1'-biphenyl]-4-yl)methyl)amino)-3-nitrobenzoate and reaction with 1-chloroethyl cyclohexyl carbonate.

3. A process for the preparation of candesartan cilexetil according to claim 1 or 2, comprising the steps of:
a') reaction between C₁₋₄alkyl 2-((*tert-*butoxycarbonyl)amino)-3-nitrobenzoate and 5-(4'-(bromomethyl)-[1,1'-biphenyl]-2-yl)-1-trityl-1H-tetrazole to give C₁₋₄alkyl 2-((tert-butoxycarbonyl)((2'-(1-trityl-1H-tetrazol-5-yl)-[1,1'-biphenyl]-4-yl)methyl)amino)-3-nitrobenzoate,
a") hydrolysis of C₁₋₄alkyl 2-((tert-butoxycarbonyl)((2'-(1-trityl-1H-tetrazol-5-yl)-[1,1'-biphenyl]-4-yl)methyl)amino)-3-nitrobenzoate to give 1-(((cyclohexyloxy)carbonyl)oxy)ethyl 2-((tert-butoxycarbonyl)((2'-(1-trityl-1H-tetrazol-5-yl)-[1,1'-biphenyl]-4-yl)methyl)amino)-3-nitrobenzoate,
a) hydrogenation of 1-(((cyclohexyloxy)carbonyl)oxy)ethyl 2-((*tert*-butoxycarbonyl)((2'-(1-trityl-1*H*-tetrazol-5-yl)-[1,1'-biphenyl]-4-yl)methyl)amino)-3-nitrobenzoate by using a catalyst in an organic solvent to get a typically oily product of 1-(((cyclohexyloxy)carbonyl)oxy)ethyl 3-amino-2-((tert-butoxycarbonyl)((2'-(1 -trityl-1H-tetrazol-5-yl)-[1,1'-biphenyl]-4-yl)methyl)amino)benzoate as specified in claim 1,
b) addition of an acid in an organic solvent to get 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, wherein the acid is HCl,
c) precipitation of 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of any pharmaceutically acceptable salt, optionally by addition of an anti-solvent,
d) ring closure with tetraethyl orthocarbonate to get candesartan cilexetil,
e) optional addition of an anti-solvent.

4. The process according to any of claims 1 to 3 wherein the organic solvent used in step a) is selected from the group consisting of an acidic alcohol or an ester such as ethyl acetate or any mixture thereof, preferably isopropyl acetate is used.

5. The process according to any of claims 1 to 4 wherein the organic solvent used in step b) is selected from the group consisting of an alcohol, ester, ether or any mixture thereof, preferably ethanol.

6. The process according to any of claims 1 to 5 wherein the anti-solvent used in step c) is *tert*-butyl methyl ether.

7. The process according to any of claims 1 to 6 wherein the molar ratio between the salt of 1-(((cyclohexyloxy)carbonyl)oxy)ethyl 2-(((2'-(1H-tetrazol-5-yl)-[1,1'-biphenyl]-4-yl)methyl)amino)-3-aminobenzoate and tetraethyl orthocarbonate is from 1 : 3 to 1 : 8.

8. The process according to any of claims 1 to 7 wherein the anti-solvent used in step e) is *tert*-butyl methy ether or THF.

9. Compound 1-(((cyclohexyloxy)carbonyl)oxy)ethyl 3-amino-2-((*tert*-butoxycarbonyl)((2'-(1-trityl-1H-tetrazol-5-yl)-[1,1'-biphenyl]-4-I)methyl)amino)benzoate or any pharmaceutically acceptable salt thereof.

10. Compound 1-(((cyclohexyloxy)carbonyl)oxy)ethyl 2-(((2'-(1*H*-tetrazol-5-yl)-[1,1'-biphenyl]-4-yl)methyl)amino)-3-aminobenzoate, which is in the form of hydrochloride salt.

11. Use of the compound 1-(((cyclohexyloxy)carbonyl)oxy)ethyl 2-(((2'-(1H-tetrazol-5-yl)-[1,1'-biphenyl]-4-yl)methyl)amino)-3-aminobenzoate in the form of hydrochloride salt according to claim 10 in the manufacture of candesartan cilexitil.

12. A process for the preparation of a pharmaceutical composition comprising the steps of preparing candesartan cilexetil by a process according to any of the preceding claims 1 to 8 followed by mixing a therapeutically effective amount thereof with one or more pharmaceutically acceptable excipient(s) and optionally with one or more other active substance(s).

## Patentansprüche

1. Verfahren zur Herstellung von Candesartancilexetil, umfassend die Schritte:
(a) Hydrierung von 1-(((Cyclohexyloxy)carbonyl)-oxy)ethyl-2-((tert-butoxycarbonyl)((2'-(1-trityl-1H-tetrazol-5-yl)-[1,1'-biphenyl]-4-yl)methyl)amino)-3-nitrobenzoat unter Verwendung eines Katalysators in einem organischen Lösungsmittel, um ein normalerweise öliges Produkt von 1-(((Cyclohexyloxy)carbonyl)oxy)-ethyl-3-amino-2-((tert-butoxycarbonyl) ((2'-(1-trityl-1H-tetrazol-5-yl)-[1,1'-biphenyl]-4-yl)methyl)amino)benzoat zu erhalten, wobei der Katalysator Raney-Ni ist,
(b) Zugabe einer Säure in einem organischen Lösungsmittel, um 1-(((Cyclohexyloxy)carbonyl)oxy)ethyl-2-(((2'-(1H-tetrazol-5-yl)-[1,1'-biphenyl]-4-yl)methyl)-amino)-3-aminobenzoat in Form irgendeines pharmazeutisch annehmbaren Salzes zu erhalten, wobei die Säure HCl ist,
(c) Ausfällen von 1-(((Cyclohexyloxy)carbonyl)-oxy)ethyl-2-(((2'-(1H-tetrazol-5-yl)-[1,1'-biphenyl]-4-yl)methyl)amino)-3-aminobenzoat in Form irgendeines pharmazeutisch annehmbaren Salzes, gegebenenfalls durch Zugabe eines Anti-Lösungsmittels, ausgewählt aus der Gruppe bestehend aus einem Ether, einem Alkan, einem chlorierten Alkan oder irgendeiner Mischung davon,
(d) Ringschluss mit Tetraethylorthocarbonat, um Candesartancilexetil zu erhalten,
(e) gegebenenfalls Zugabe eines Anti-Lösungsmittels, wobei das Anti-Lösungsmittel aus der Gruppe bestehend aus einem Ether, wie THF, tert-Butylmethylether, Methyl-THF, Diisopropylether und Dioxan, einem Ester, wie Ethylacetat und Isopropylacetat, oder irgendeiner Mischung davon ausgewählt ist.

2. Verfahren zur Herstellung von Candesartancilexetil gemäss Anspruch 1, wobei 1-(((Cyclohexyloxy)carbonyl)-oxy)ethyl-2-((tert-butoxycarbonyl)((2'-(1-trityl-1H-tetrazol-5-yl)-[1,1'-biphenyl]-4-yl)methyl)amino)-3-nitrobenzoat durch Hydrolyse von Ethyl-2-((tert-butoxycarbonyl)((2'-(1-trityl-1H-tetrazol-5-yl)-[1,1'-biphenyl]-4-yl)methyl)amino)-3-nitrobenzoat und Reaktion mit 1-Chlorethylcyclohexylcarbonat hergestellt wird.

3. Verfahren zur Herstellung von Candesartancilexetil gemäss Anspruch 1 oder 2, umfassend die Schritte:
(a') Reaktion zwischen C₁₋₄-Alkyl-2-((tert-butoxycarbonyl)amino)-3-nitrobenzoat und 5-(4'-(Brommethyl)-[1,1'-biphenyl]-2-yl)-1-trityl-1H-tetrazol, um C₁₋₄-Alkyl-2-((tert-butoxycarbonyl)((2'-(1-trityl-1H-tetrazol-5-yl)-[1,1'-biphenyl]-4-yl)methyl)amino)-3-nitrobenzoat zu erhalten,
(a'') Hydrolyse von C₁₋₄-Alkyl-2-((tert-butoxycarbonyl)((2'-(1-trityl-1H-tetrazol-5-yl)-[1,1'-biphenyl]-4-yl)methyl)amino)-3-nitrobenzoat, um 1-(((Cyclohexyloxy)carbonyl)oxy)ethyl-2-((tert-butoxycarbonyl)((2'-(1-trityl-1H-tetrazol-5-yl)-[1,1'-biphenyl]-4-yl)methyl)amino)-3-nitrobenzoat zu erhalten,
(a) Hydrierung von 1-(((Cyclohexyloxy)carbonyl)-oxy)ethyl-2-((tert-butoxycarbonyl)((2'-(1-trityl-1H-tetrazol-5-yl)-[1,1'-biphenyl]-4-yl)methyl)amino)-3-nitrobenzoat unter Verwendung eines Katalysators in einem organischen Lösungsmittel, um ein normalerweise öliges Produkt von 1-(((Cyclohexyloxy)carbonyl)oxy)ethyl-3-amino-2-((tert-butoxycarbonyl)((2'-(1-trityl-1H-tetrazol-5-yl)-[1,1'-biphenyl]-4-yl)methyl)amino)benzoat, wie in Anspruch 1 spezifiziert, zu erhalten,
(b) Zugabe einer Säure in einem organischen Lösungsmittel, um 1-(((Cyclohexyloxy)carbonyl)oxy)ethyl-2-(((2'-(1H-tetrazol-5-yl)-[1,1'-biphenyl]-4-yl)methyl)-amino)-3-aminobenzoat in Form irgendeines pharmazeutisch annehmbaren Salzes zu erhalten, wobei die Säure HCl ist,
(c) Ausfällen von 1-(((Cyclohexyloxy)carbonyl)-oxy)ethyl-2-(((2'-(1H-tetrazol-5-yl)-[1,1'-biphenyl]-4-yl)methyl)amino)-3-aminobenzoat in Form irgendeines pharmazeutisch annehmbaren Salzes gegebenenfalls durch Zugabe eines Anti-Lösungsmittels,
(d) Ringschluss mit Tetraethylorthocarbonat, um Candesartancilexetil zu erhalten,
(e) gegebenenfalls Zugabe eines Anti-Lösungsmittels.

4. Verfahren gemäss einem der Ansprüche 1 bis 3, wobei das in Schritt (a) verwendete organische Lösungsmittel aus der Gruppe bestehend aus einem sauren Alkohol oder einem Ester, wie Ethylacetat, oder irgendeiner Mischung davon ausgewählt ist, wobei vorzugsweise Isopropylacetat verwendet wird.

5. Verfahren gemäss einem der Ansprüche 1 bis 4, wobei das in Schritt (b) verwendete organische Lösungsmittel aus der Gruppe bestehend aus einem Alkohol, Ester, Ether oder irgendeiner Mischung davon, vorzugsweise Ethanol, ausgewählt ist.

6. Verfahren gemäss einem der Ansprüche 1 bis 5, wobei das in Schritt (c) verwendete Anti-Lösungsmittel tert-Butylmethylether ist.

7. Verfahren gemäss einem der Ansprüche 1 bis 6, wobei das Molverhältnis zwischen dem Salz von 1-(((Cyclohexyloxy)-carbonyl)oxy)ethyl-2-(((2'-(1H-tetrazol-5-yl)-[1,1'-biphenyl]-4-yl)methyl)amino)-3-aminobenzoat und Tetraethylorthocarbonat 1:3 bis 1:8 beträgt.

8. Verfahren gemäss einem der Ansprüche 1 bis 7, wobei das in Schritt (e) verwendete Anti-Lösungsmittel tert-Butylmethylether oder THF ist.

9. Verbindung 1-(((Cyclohexyloxy)carbonyl)oxy)ethyl-3-amino-2-((tert-butoxycarbonyl)((2'-(1-trityl-1H-tetrazol-5-yl)-[1,1'-biphenyl]-4-1)methyl)amino)benzoat oder irgendein pharmazeutisch annehmbares Salz davon.

10. Verbindung 1-(((Cyclohexyloxy)carbonyl)oxy)ethyl-2-(((2'-(1H-tetrazol-5-yl)-[1,1'-biphenyl]-4-yl)methyl)-amino)-3-aminobenzoat, die in Form eines Hydrochloridsalzes vorliegt.

11. Verwendung der Verbindung 1-(((Cyclohexyloxy)carbonyl)-oxy)ethyl-2-(((2'-(1H-tetrazol-5-yl)-[1,1'-biphenyl]-4-yl)methyl)amino)-3-aminobenzoat in Form eines Hydrochloridsalzes gemäss Anspruch 10 bei der Herstellung von Candesartancilexitil.

12. Verfahren zur Herstellung einer pharmazeutischen Zusammensetzung, umfassend die Schritte: Herstellung von Candesartancilexetil durch ein Verfahren gemäss irgendeinem der vorhergehenden Ansprüche 1 bis 8, gefolgt von Mischen einer therapeutisch wirksamen Menge davon mit einem oder mehreren pharmazeutisch annehmbaren Hilfsstoff(en) und gegebenenfalls mit einem oder mehreren anderen Wirkstoff(en).

## Revendications

1. Procédé de préparation de candésartan cilexétil comprenant les étapes de :
a) hydrogénation de 2-((*tert*-butoxycarbonyl)((2'-(1-trityl-1*H*-tétrazol-5-yl)-[1,1'-biphényl]-4-yl)méthyl)amino)-3-nitrobenzoate de 1-(((cyclohexyloxy)carbonyl)oxy)éthyle en utilisant un catalyseur dans un solvant organique pour obtenir un produit typiquement huileux de 3-amino-2-((tert-butoxycarbonyl)((2'-(1-trityl-1H-tétrazol-5-yl)-[1,1'-biphényl]-4-yl)méthyl)amino)benzoate de 1-(((cyclohexyloxy)carbonyl)oxy)éthyle, dans lequel le catalyseur est du Ni Raney,
b) addition d'un acide dans un solvant organique pour obtenir du 2-(((2'-(1H-tétrazol-5-yl)-[1,1'-biphényl]-4-yl)méthyl)amino)-3-aminobenzoate de 1-(((cyclohexyloxy)carbonyl)oxy)éthyle sous la forme de n'importe quel sel pharmaceutiquement acceptable, dans lequel l'acide est HCl,
c) précipitation du 2-(((2'-(1H-tétrazol-5-yl)-[1,1'-biphényl]-4-yl)méthyl)amino)-3-aminobenzoate de 1-(((cyclohexyloxy)carbonyl)oxy)éthyle sous la forme de n'importe quel sel pharmaceutiquement acceptable, facultativement par addition d'un anti-solvant choisi dans le groupe constitué d'un éther, un alcane, un alcane chloré ou n'importe quel mélange de ceux-ci,
d) fermeture de cycle avec de l'orthocarbonate de tétraéthyle pour obtenir du candésartan cilexétil,
e) addition facultative d'un anti-solvant, dans lequel l'anti-solvant est choisi dans le groupe constitué d'un éther tel que THF, l'éther méthyl-*tert*-butylique, le méthyl-THF, l'éther di-isopropylique et le dioxane, un ester tel que l'acétate d'éthyle et l'acétate d'isopropyle ou n'importe quel mélange de ceux-ci.

2. Procédé de préparation de candésartan cilexétil selon la revendication 1, dans lequel le 2-((*tert*-butoxycarbonyl)((2'-(1-trityl-1H-tétrazol-5-yl)-[1,1'-biphényl]-4-yl)methyl)amino)-3-nitrobenzoate de 1-(((cyclohexyloxy)carbonyl)oxy)éthyle est préparé par hydrolyse de 2-((*tert*-butoxycarbonyl)((2'-(1-trityl-1H-tetrazol-5-yl)-[1,1'-biphényl]-4-yl)methyl)amino)-3-nitrobenzoate d'éthyle et réaction avec du cyclohexyl-carbonate de 1-chloroéthyle.

3. Procédé de préparation de candésartan cilexétil selon la revendication 1 ou 2, comprenant les étapes de :
a') réaction entre du 2-((*tert*-butoxycarbonyl)amino)-3-nitrobenzoate d'alkyle en C_{1 à 4} et du 5-(4'-(bromométhyl)-[1,1'-biphényl]-2-yl)-1-trityl-1H-tétrazole pour donner du 2-((*tert*-butoxycarbonyl)((2'-(1-trityl-1H-tétrazol-5-yl)-[1,1'-biphenyl]-4-yl)méthyl)amino)-3-nitrobenzoate d'alkyle en C_{1 à 4},
a") hydrolyse de 2-((tert-butoxycarbonyl)((2'-(1-trityl-1H-tétrazol-5-yl)-[1,1'-biphényl]-4-yl)méthyl)amino)-3-nitrobenzoate d'alkyle en C_{1 à 4} pour donner du 2-((tert-butoxycarbonyl)((2'-(1-trityl-1H-tétrazol-5-yl)-[1,1'-biphényl]-4-yl)méthyl)amino)-3-nitrobenzoate de 1-(((cyclohexyloxy)carbonyl)oxy)éthyle,
a) hydrogénation de 2-((*tert*-butoxycarbonyl)((2'-(1-trityl-1*H*-tétrazol-5-yl)-[1,1'-biphényl]-4-yl)méthyl)amino)-3-nitrobenzoate de 1-(((cyclohexyloxy)carbonyl)oxy)éthyle en utilisant un catalyseur dans un solvant organique pour obtenir un produit typiquement huileux de 3-amino-2-((tert-butoxycarbonyl)((2'-(1-trityl-1H-tétrazol-5-yl)-[1,1'-biphényl]-4-yl)méthyl)amino)benzoate de 1-(((cyclohexyloxy)carbonyl)oxy)éthyle, comme spécifié dans la revendication 1,
b) addition d'un acide dans un solvant organique pour obtenir du 2-(((2'-(1H-tétrazol-5-yl)-[1,1'-biphényl]-4-yl)méthyl)amino)-3-aminobenzoate de 1-(((cyclohexyloxy)carbonyl)oxy)éthyle sous la forme de n'importe quel sel pharmaceutiquement acceptable, dans lequel l'acide est HCl,
c) précipitation de 2-(((2'-(1H-tétrazol-5-yl)-[1,1'-biphényl]-4-yl)méthyl)amino)-3-aminobenzoate de 1-(((cyclohexyloxy)carbonyl)oxy)éthyle sous la forme de n'importe quel sel pharmaceutiquement acceptable, facultativement par addition d'un anti-solvant,
d) fermeture de cycle avec de l'orthocarbonate de tétraéthyle pour obtenir du candésartan cilexétil,
e) addition facultative d'un anti-solvant.

4. Procédé selon l'une quelconque des revendications 1 à 3 dans lequel le solvant organique utilisé à l'étape a) est choisi dans le groupe constitué d'un alcool acide ou d'un ester tel que l'acétate d'éthyle ou n'importe quel mélange de ceux-ci, de préférence de l'acétate d'isopropyle est utilisé.

5. Procédé selon l'une quelconque des revendications 1 à 4 dans lequel le solvant organique utilisé à l'étape b) est choisi dans le groupe constitué d'un alcool, un ester, un éther ou n'importe quel mélange de ceux-ci, de préférence de l'éthanol.

6. Procédé selon l'une quelconque des revendications 1 à 5, dans lequel l'anti-solvant utilisé à l'étape c) est l'éther méthyl *tert*-butylique.

7. Procédé selon l'une quelconque des revendications 1 à 6 dans lequel le rapport molaire entre le sel de 2-(((2'-(1H-tétrazol-5-yl)-[1,1'-biphényl]-4-yl)méthyl)amino)-3-aminobenzoate de 1-(((cyclohexyloxy)carbonyl)oxy)éthyle et l'orthocarbonate de tétraéthyle va de 1:3 à 1:8.

8. Procédé selon l'une quelconque des revendications 1 à 7, dans lequel l'anti-solvant utilisé à l'étape e) est l'éther méthyl *tert*-butylique ou le THF.

9. Composé 3-amino-2-((*tert*-butoxycarbonyl)((2'-(1-trityl-1H-tétrazol-5-yl)-[1,1'-biphényl]-4-I)méthyl)amino)benzoate de 1-(((cyclohexyloxy)carbonyl)oxy)éthyle ou n'importe quel sel de celui-ci pharmaceutiquement acceptable.

10. Composé 2-(((2'-(1*H*-tétrazol-5-yl)-[1,1'-biphényl]-4-yl)méthyl)amino)-3-aminobenzoate de 1-(((cyclohexyloxy)carbonyl)oxy)éthyle, qui est sous la forme de sel chlorhydrate.

11. Utilisation du composé 2-(((2'-(1H-tétrazol-5-yl)-[1,1'-biphényl]-4-yl)méthyl)amino)-3-aminobenzoate de 1-(((cyclohexyloxy)carbonyl)oxy)éthyle sous la forme de sel chlorhydrate selon la revendication 10 dans la fabrication de candésartan cilexétil.

12. Procédé de préparation d'une composition pharmaceutique comprenant les étapes de préparation de candésartan cilexétil par un procédé selon l'une quelconque des revendications précédentes 1 à 8 suivies par le mélange d'une quantité thérapeutiquement efficace de celui-ci avec un ou plusieurs excipient(s) pharmaceutiquement acceptable(s) et facultativement avec une ou plusieurs autres substance(s) active(s).
